(19) Europäisches Patentamt
European Patent Office
Office européen des brevets

(11) Numéro de publication: **0 167 459 B1**

(12) # FASCICULE DE BREVET EUROPEEN

(45) Date de publication de fascicule du brevet: **06.11.91**

(51) Int. Cl.[5]: **C07D 263/28**, A61K 31/42

(21) Numéro de dépôt: **85401360.4**

(22) Date de dépôt: **04.07.85**

(54) **2-amino oxazolines et leur procédé d'obtention.**

(30) Priorité: **05.07.84 CH 3263/84**

(43) Date de publication de la demande:
**08.01.86 Bulletin 86/02**

(45) Mention de la délivrance du brevet:
**06.11.91 Bulletin 91/45**

(84) Etats contractants désignés:
**AT BE CH DE FR GB IT LI LU NL SE**

(56) Documents cités:
**EP-A- 0 117 961**
**FR-A- 2 444 673**
**FR-M- 2 448**

(73) Titulaire: **Albert ROLLAND S.A. Société dite**
**49, Rue St-André-des-Arts**
**F-75006 Paris(FR)**

(72) Inventeur: **Schaefer, Michel**
**4, Rue François Girardon**
**F-91380 Chilly Mazarin(FR)**
Inventeur: **Moinet, Gérard**
**15, Rue Lamartine**
**F-91400 Orsay(FR)**
Inventeur: **Bessin, Pierre**
**1, Allée Bossuet**
**F-91380 Chilly Mazarin(FR)**

(74) Mandataire: **Burtin, Jean-François**
**Cabinet GEFIB, 59, rue Edouard-Vaillant**
**F-92300 Levallois-Perret(FR)**

**EP 0 167 459 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

Dans la demande de brevet européen EP-A-0117 961, la demanderesse a décrit à titre de composés nouveaux, les composés hétéro-cycliques dont la structure cyclique comporte deux hétéro-atomes, identiques ou différents, substitués par un groupe amino libre ou substitué.
Ces composés hétéro-cycliques répondent à la formule générale I'

(I')

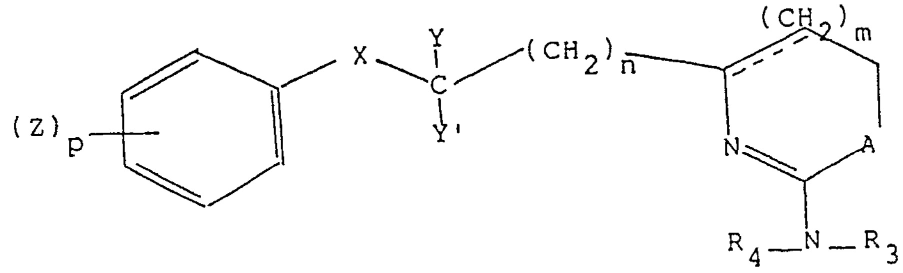

dans laquelle Z est de l'hydrogène, un atome d'halogène, un radical alcoyle inférieur, un radical alcoxy inférieur, un radical trifluorométhyle, un radical trifluoro méthoxy, cyano, nitro, carboxamido, un radical alcényle inférieur, un radical (alcoyle inférieur) thio ou un groupe alcoylène dioxy.
X représente de l'oxygène, du soufre, un radical imino de formule N-$R_1$, dans laquelle $R_1$ est de l'hydrogène ou un radical alcoyle inférieur, un radical méthylène ou une liaison directe
Y représente de l'hydrogène, un radical alcoyle inférieur, un radical phényle non substitué ou substitué, un hydroxyle ou un radical phénoxy
Y' représente de l'hydrogène
ou bien Y et Y' forment ensemble l'oxygène d'un groupe carbonyle
ou bien Y forme avec le radical phényle adjacent une structure bicyclique homocyclique ou hétérocyclique, saturée ou non saturée A représente S ou NH
$R_3$ et $R_4$ représentent de l'hydrogène, un radical alcoyle inférieur, alcenyle inférieur, aralcoyle inférieur ou (hétéroaryle) alcoyle inférieur, aryle, alcoloxy carbonyle acyle dérivé d'un acide organique carboxylique ayant de 1 à 10 atomes de carbone
ou bien $R_3$ et $R_4$ forment avec l'atome d'azote auxquels ils sont liés, la chaine alcoylène d'une structure hétérocyclique azotée, éventuellement interrompue par un ou deux autres hétéro-atomes
m est égal à O ou 1
et p est égal à 1, 2 ou 3

L'invention se rapportait aussi aux sels de composés de formule générale I' dans laquelle les substituants Z, X, Y, Y', A, n et p sont définis comme précédemment, avec un acide minéral ou organique, de préférence thérapeutiquement compatible.

La présente inventon a pour objet des composés proches de ceux de ladite demande de brevet européen, à savoir les oxazolines de formule générale I

(I)

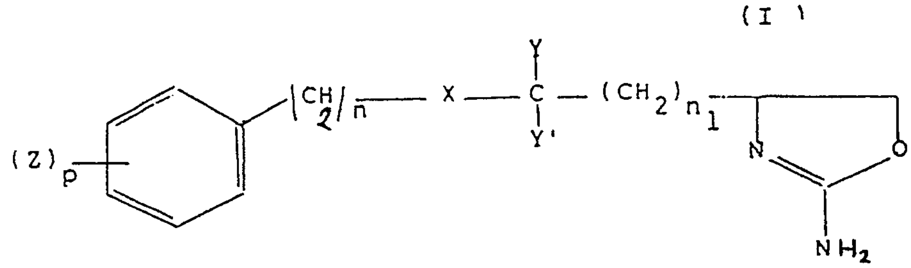

dans laquelle Z est de l'hydrogène, un atome d'halogène, un radical alcoxy ayant de 1 à 6 atomes de Carbone
X représente de l'oxygène, du soufre, un radical imino, un radical méthylène ou une liaison directe

Y représente de l'hydrogène
ou bien Y et Y' forment ensemble l'oxygène d'un groupe carbonyle
ou bien Y' forme avec le radical phényle adjacent, lorsque n est égal à zéro, une structure bicyclique, hétérocyclique, saturée ou non saturée, choisie dans le groupe constitué par le benzofuran, l'indole, l'indoline et le dihydrobenzofuran
n est égal à zéro ou 1
$n_1$ est égal à O,1 ou 2
et p est égal à 1,2 ou 3
sous forme racémique ou optiquement active.

Les composés selon l'invention peuvent être salifiés par addition d'un acide minéral ou organique, de préférence un acide thérapeutiquement compatible.

Les composés selon l'invention comportent au moin un atome de carbone chiral et peuvent, de ce fait, exister sous forme racémique ou, après dédoublement, sous une forme optiquement-active.

Parmi les acides physiologiquement compatibles, on pourra citer les chlorhydrates, bromhydrates, les sulfates, les nitrates, les phosphates, les sulfites, les acétates, les butyrates, les caproates, les subérates, les succinates, les tartrates, les citrates, les itaconates, les glutamates, les aspartates, les benzoates, les triméthoxybenzoates, les salicylates, les niflumates, les flufénamates, les méfénamates, les nicotinates, les isonicotinates, les benzene-sulfonates, les méthanesulfonates, les éthanesulfonates, les iséthionates, les paratoluène sulfonates, les naphtalène-sulfonates, les glucose 1-phosphates ou les glucoses 1, 6-diphosphates.

Les acides qui ne sont pas utilisables en thérapeutique peuvent servir comme moyen d'isolement, de purification ou de dédoublement.

On pourra citer, à cet égard, les perchlorates, les iodates, les bromates, les vanadates ou les chromates, les sels avec l'acide strychnique, l'acide d-chrysanthémique, l'acide indolyl-3 acétique, l'acide dichlorophénoxy-isobutyrique ou l'acide citraconique.

Pour autant que l'invention soit concernée, le terme alcoyle inférieur ayant de 1 à 6 atomes de carbone désigne un radical hydrocarboné en chaine linéaire ou ramifiée. Des exemples de ceux-ci sont butyle, <u>ter</u> butyle, néopentyle, n-héxyle, méthyle ou éthyle.

Un radical alcoxy inférieur comporte un radical alcoyle défini comme précédemment.

Parmi les halogènes, on citera plus particulièrement le fluor ou le chlore. Néanmoins, les dérivés bromés et iodés sont d'un intérêt équivalent.

Lorsque Y' forme avec le radical phényle adjacent à une structure bicyclique, les composés formés répondent à la formule générale $I_E$

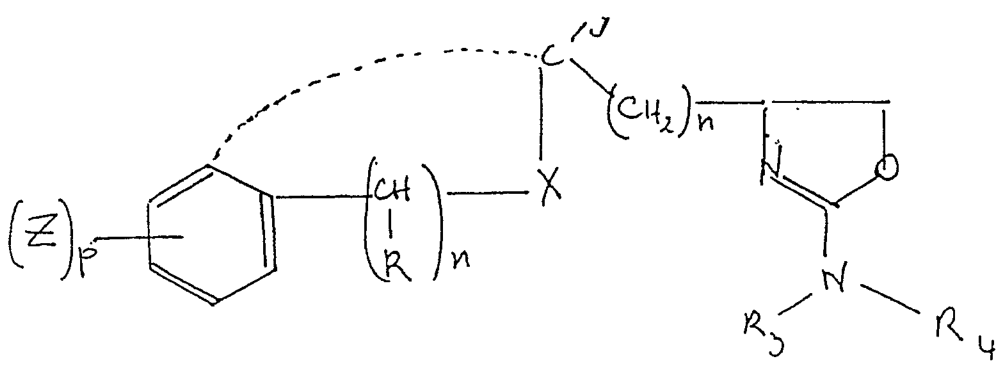

$(I_E)$

et sont des dérivés de l'indole de formule

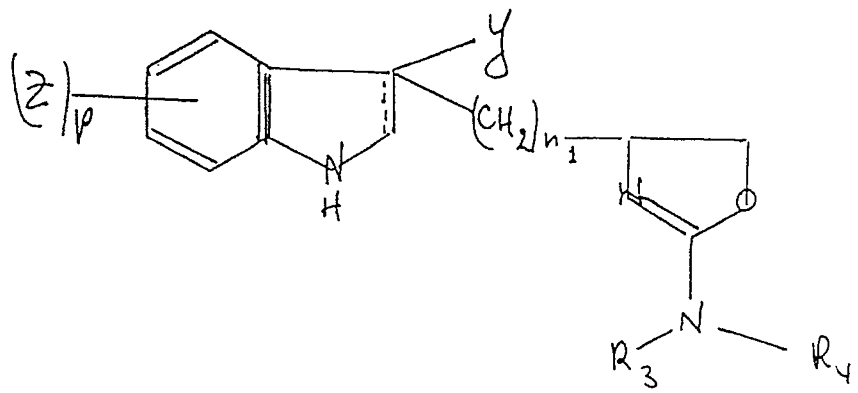

ou des dérivés du benzofuran de formule

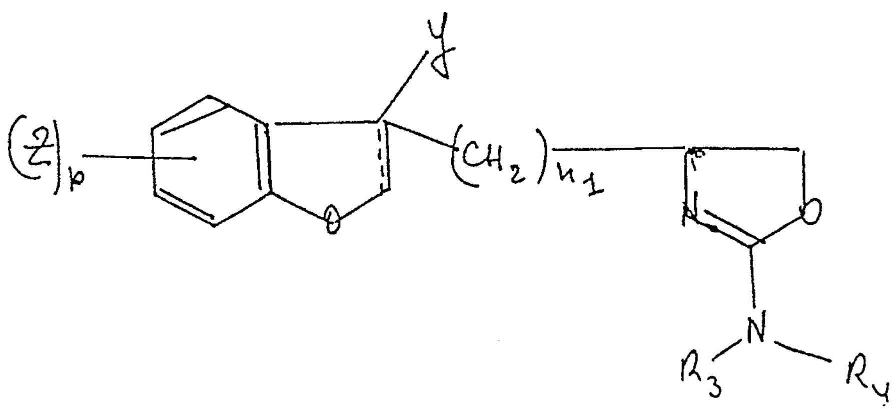

et dans ce deux cas, Y représente de l'hydrogène ou forme avec le carbone adjacent, une double liaison et les autres substituants sont définis comme ci-dessus.

L'invention a également pour objet un procédé d'obtention des composés de formule générale I :

(I)

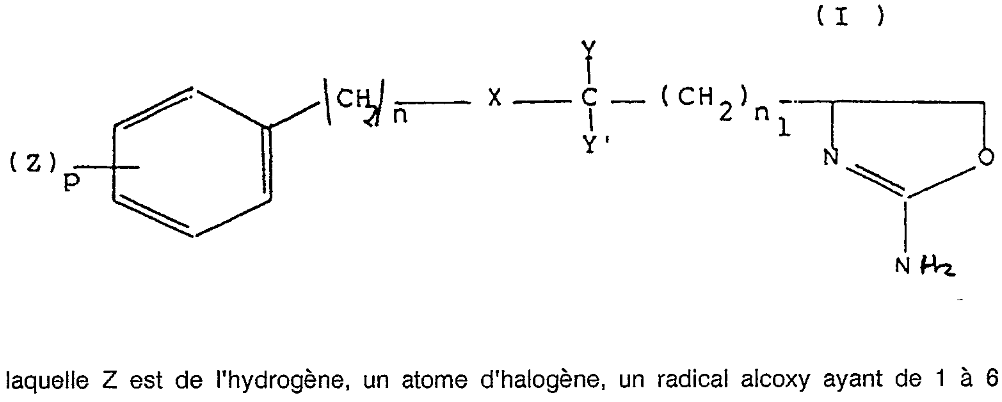

dans laquelle Z est de l'hydrogène, un atome d'halogène, un radical alcoxy ayant de 1 à 6 atomes de Carbone
X représente de l'oxygène, du soufre, un radical imino, un radical méthylène ou une liaison directe
Y représente de l'hydrogène
Y' représente de l'hydrogène
ou bien Y et Y' forment ensemble l'oxygène d'un groupe carbonyle
ou bien Y forme avec le radical phényle adjacent, lorsque n est égal à zéro, une structure bicyclique hétérocyclique, saturée ou non saturée choisie dans le groupe constitué par le benzofuran, l'indole l'indoline et le dihydrobenzofuran
n est égal à zéro ou 1
$n_1$ est égal à O, 1 ou 2

et p est égal à 1, 2 ou 3
qui consiste en ce que l'on réduit un dérivé aminé de formule générale II

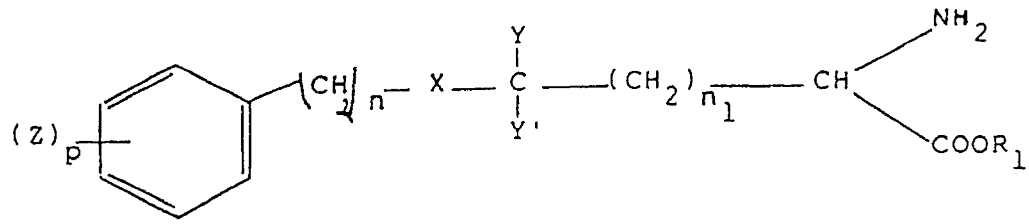

(II)

dans laquelle les substituants Z, p, n, X, Y, Y' et $n_1$ sont définis comme ci-dessus et $R_1$ est un radical alcoyle inférieur ou de l'hydrogène par un hydrure mixte de métal alcalin, en un amino-alcool de formule générale III

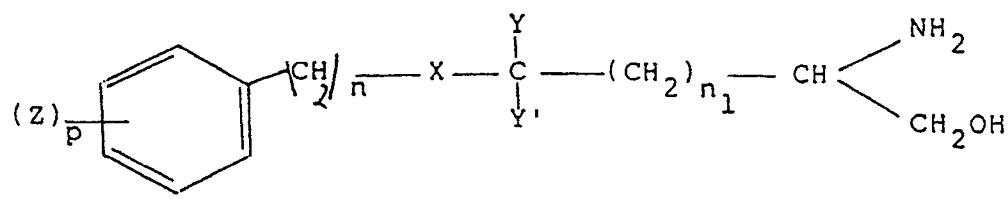

(III)

dans laquelle les substituants Z, p, n, X, Y, Y' et $n_1$ sont définis comme ci-dessus
puis par action d'un halogénure de cyanogène en présence d'un agent alcalin, le transforme en une amino-oxazoline de formule I

Le procédé selon l'invention peut également être défini par les modes opératoires suivants, actuellement préférés :

- l'hydrure mixte de métal alcalin est un aluminohydrure de sodium ou de lithium
- la réduction est effectuée dans un solvant oxygéné comme un éther cyclique ou linéaire
- l'halogénure de cyanogène est le bromure de cyanogène
- l'agent alcalin est un acétate de métal alcalin et particulièrement l'acétate de sodium.

Les esters d'amino acides de formule générale II sont obtenus à partir des aldehydes correspondants par réaction de Strecker, hydrolyse de la cyano-amine formée en amino-acide puis estérification (cf. R. Sola et al. Tetrahedron Letters 14 (1983) 1501).

En particulier, le benzyloxy propanoate d'éthyle est préparé à partir du dibromopropionate d'éthyle selon le procédé décrit par W. GRASSMAN (Chem. Ber. 91 (1958) 538).

Le 4-(benzofuranyl-3) 2-aminoacétate d'éthyle est un produit nouveau. Il est obtenu selon un procédé semblable.

Les 2-amino-oxazolines selon l'invention se caractérisent par des propriétés pharmacologiques intéressantes, et notamment par des propriétés anti-dépressives, hypertensives et analeptiques vasculaires. Elles agissent sur les récepteurs $\alpha_2$, soit par un phénomène de bloquage, soit par un effet agoniste. Ces molécules présentent un intérêt particulier par le fait qu'à faibles doses (100 à 500 mcg/kg par voie intraveineuse chez le chien anesthésié), elles manifestent une activité $\alpha_2$-bloquante qui se révèle par la suppression des vaso-constrictions primaires sous l'influence de la Dopamine, sans en altérer les vaso-dilatations secondaires (effet biphasique). Cet effet s'apparente donc à celui de la Yohimbine.

Une publication récente de J.A SCOTT et F.T CREWS, (J. Ph. Exp. Th. 224 (1982) 640), a montré qu'un traitement combiné d'un anti-dépresseur et d'un antagoniste $\alpha_2$ provoquait une diminution rapide de la densité des récepteurs post-synaptiques (en 1 jour environ). Ce phénomène joue un rôle important, en particulier sur l'action des anti-dépresseurs en médecine humaine, en raccourcissant le temps de latence de leur efficacité. Il en résulte une plus grande rapidité d'action des produits de l'invention en tant qu'agent antidépresseur.

En raison de leurs propriétés pharmacologiques, les composés selon l'invention trouvent un emploi en thérapeutique comme médicament antidépresseur ou comme médicament antihypertenseur.

A ces fins, ils sont employés sous forme de compositions pharmaceutiques qui renferment à titre de

principe actif, au moins un composé de formule I sous forme racémique ou optiquement-active, ou un de ses sels d'addition avec un acide minéral ou organique, en association ou en mélange avec un excipient ou un véhicule inerte non-toxique, pharmaceutiquement-acceptable.

Les composés selon l'invention sont destinés à être administrés de préférence par voie parentérale, buccale ou rectale.

Les formes pharmaceutiques selon l'invention sont donc celles qui conviennent pour l'administration par l'une de ces voies comme, par exemple, les comprimés nus ou enrobés, les dragées, les pilules, les gélules, les solutés ou suspensions injectables, les gouttes, les sirops, les solutions buvables, les préparations lyophilisées pour injection, les suppositoires.

La posologie pourra varier dans de larges proportions en fonction de la voie d'administration, de l'indication thérapeutique, de l'âge et du poids du sujet, de l'ancienneté de la maladie à traiter et, éventuellement, de la nature du composé de formule générale I.

En règle générale, la posologie unitaire s'échelonne de O,1 mg à 250 mg et la posologie journalière varie chez l'adulte de 0,1 mg à 500 mg. La posologie unitaire par voie parentérale sera de préférence de 0,1 mg à 100 mg et la posologie unitaire par voie digestive sera de préférence de 0,2 mg à 250 mg.

Les exemples suivants illustrent l'invention.

EXEMPLE 1

Stade A

3-benzyloxy-2-amino-propanol-1

A 5 g (2,2 mmoles) de 3-benzyloxy-2-amino-propanoate d'éthyle dans 100 ml d'éther éthylique sec, on ajoute 1,7 g (4,4 mmoles) d'alumino hydrure de lithium sous argon. Après 2 heures d'agitation à température ambiante, on ajoute par portions 10 g de bouillie de sulfate de sodium sec dans un peu d'eau. Le mélange résultant est filtré, broyé, lavé plusieurs fois au chlorure de méthylène. Le filtrat est lavé deux fois à l'eau et séché. Après évaporation du solvant, on recueille 3,69 g d'amino alcool. Rendement = 91%.

Stade B

4-(benzyloxy-méthyl) 2-amino-oxazoline

A 3,69 g (2 mmoles) de 3-benzyloxy-2-amino-propanol-1 dans 500 ml de méthanol anhydre, on ajoute 3,3 g (4 mmoles) d'acétate de sodium sec. Sous argon on ajoute, goutte à goutte, 2g2 (2 mmoles) de bromure de cyanogène dissout dans 6 ml de méthanol anhydre. Après 4 heures d'agitation à température ambiante, le méthanol est évaporé, le résidu est repris par 50 ml d'acétate d'éthyle et lavé avec 10 ml d'eau.

La phase organique est séchée puis évaporée. On recueille 1,95 g de 2-amino-4-(benzyloxy-méthyl)-oxazoline. Rendement = 46%.

Le chlorhydrate est obtenu en traitant la base obtenue par une solution d'éther chlorhydrique.

Le spectre RMN et le spectre IR permettent d'établir la structure et de confirmer la substitution en position 4 et non pas en position 5.

4-(benzyloxy-méthyl ) 2-amino-oxazoline.

Poids moléculaire de la base : 206,247

PF du Chlorhydrate : 128°

| Analyse | C | H | N% |
|---|---|---|---|
| Calculé | 54,44 | 6,23 | 11,54 |
| Trouvé | 54,56 | 6,12 | 11,42 |

Les composés suivants ont été préparés par la même méthode :

EXEMPLE 2

4-phénoxy-méthyl 2-amino-oxazoline

Poids moléculaire de la base : 192,22

P F du bromhydrate : 160 °

| Analyse | C | H | N% |
|---|---|---|---|
| Calculé<br>Trouvé | 43,97<br>43,78 | 4,79<br>4,80 | 10,25<br>10,26 |

EXEMPLE 3

4-(2-chlorophénoxy méthyl ) 2-amino-oxazoline.
Poids moléculaire de la base : 226,66
P F du fumarate : 172 °

| Analyse | C | H | N% |
|---|---|---|---|
| Calculé<br>Trouvé | 49,06<br>49,23 | 4,41<br>4,50 | 8,17<br>8,27 |

EXEMPLE 4

4-(4-méthoxyphenoxy methyl ) 2-amino-oxazoline.
Poids moléculaire de la base : 222,246
P F du fumarate : 134 °

| Analyse | C | H | N % |
|---|---|---|---|
| Calculé<br>Trouvé | 51,07<br>50,96 | 5,84<br>5,77 | 10,82<br>10,72 |

| Ex | Z | p. | n | X | Y/Y' |
|---|---|---|---|---|---|
| 5 | Cl(2) | 1 | zéro | O | $H_2$ |
| 6 | H | | zéro | O | $H_2$ |
| 7 | $CH_3O$(4) | 1 | zéro | O | $H_2$ |
| 8 | H | | 1 | O | $H_2$ |
| 9 | H | | zéro | O | $H_2$ |
| 10 | Cl(4) | 1 | zéro | liaison directe | $H_2$ |
| 11 | cl(2) | 1 | zéro | " | $H_2$ |
| 12 | H (isomère +) | | zéro | " | $H_2$ |
| 13 | H | | zéro | $CH_2$ | $H_2$ |
| 14 | H (isomère -) | | zéro | $CH_2$ | $H_2$ |
| 15 | $CH_3O$ (2) | 1 | zéro | $CH_2$ | $H_2$ |
| 16 | $CH_3O$ (4) | 1 | zéro | $CH_2$ | $H_2$ |
| 17 | $CH_3O$ (3,4) | 2 | zéro | $CH_2$ | $H_2$ |
| 18 | H | | zéro | NH | Reste alcoylène* |
| 19 | Dichloro (2,3) Methoxy (4) | 3 | zéro | liaison directe | $H_2$ |
| 20 | Hydroxy (4) | 1 | zéro | " | $H_2$ |
| 21 | Dichloro (2,6) | 2 | zéro | liaison directe | $H_2$ |
| 22 | Chlore (2) isomère levogyre | 1 | zéro | " | $H_2$ |
| 23 | Chlore (2) isomère dextrogyre | 1 | zéro | " | $H_2$ |

*Reste alcoylène d'un noyau pyrrole accolé au noyau benzenique

| $n_1$ | | | PM (base) | PF°C (Sel) HCL | ANALYSE CENTESIMALE Calculée C | H | N | Trouvée C | H | N % |
|---|---|---|---|---|---|---|---|---|---|---|
| zéro | | | 226,6 | 172(1) | 49,06 | 4,41 | 8,17 | 49,23 | 4,5 | 8,27 |
| zéro | | | 192,22 | 160(2) | 43,97 | 4,79 | 10,25 | 43,78 | 4,80 | 10,26 |
| zéro | | | 222,24 | 134 | 51,07 | 5,84 | 10,82 | 50,96 | 5,77 | 10,72 |
| zéro | | | 206,24 | 128 | 54,44 | 6,23 | 11,54 | 54,56 | 6,12 | 11,42 |
| zéro | | | 176,22 | 120/122 | 56,48 | 6,16 | 13,17 | 56,35 | 6,08 | 13,05 |
| zéro | | | 210,66 | 164 | 48,6 | 4,89 | 11,34 | 48,48 | 4,99 | 11,24 |
| zéro | | | 210,66 | 158-160 | 48,6 | 4,89 | 11,34 | 48,72 | 4,99 | 11,40 |
| zéro | | | 176,22 | 128 | 56,48 | 6,16 | 13,17 | 56,62 | 6,30 | 13,23 |
| zéro | | | 190,24 | 137(2) | 48,72 | 5,57 | 10,33 | 48,79 | 5,60 | 10,22 |
| zéro | | | 176,22 | 120 | 56,48 | 6,16 | 13,17 | 56,39 | 6,25 | 13,15 |
| zéro | | | 206,24 | 136 | 54,44 | 6,23 | 11,54 | 54,35 | 6,31 | 11,38 |
| zéro | | | 206,24 | 162 | 54,44 | 6,23 | 11,54 | 54,61 | 6,37 | 11,32 |
| zéro | | | 236,27 | 173 | 52,85 | 6,28 | 10,27 | 52,60 | 6,33 | 10,09 |
| zéro | | | 215,25 | 202 | 61,53 | 5,53 | 15,38 | 61,36 | 5,46 | 15,24 |
| zéro | | | 275,14 | 200 | 42,4 | 4,2 | 8,99 | 42,22 | 4,25 | 8,99 |
| zéro | | | 192,22 | 164 | 52,52 | 5,73 | 12,25 | 52,35 | 5,86 | 12,26 |
| zéro | | | 245,11 | 172 | 42,66 | 3,94 | 9,95 | 42,80 | 4,05 | 9,87 |
| zéro | | | 210,66 | 126 | 48,60 | 4,89 | 11,34 | 48,45 | 4,99 | 11,33 |
| zéro | | | 210,66 | 129 | 48,60 | 4,89 | 11,34 | 48,45 | 4,96 | 11,33 |

1 - Fumarate
2 - Bromhydrate

Exemple de préparation des énantiomères des composés selon l'invention

Stade A

(O-chloro phényl)-3-amino-2-propanol-1 optiquement actif

a) N-acylation :

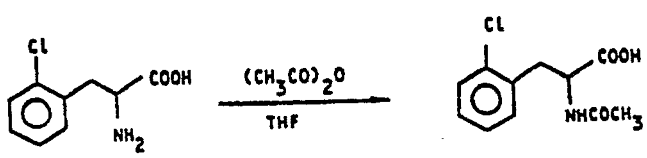


Une suspension de 60 g (0,30 M) d'ortho-chloro-phényl alanine, dans 1 1 de tetrahydrofurane est agitée une nuit à température ambiante ; on ajoute alors 75 cm$^3$ d'eau, puis on évapore le THF sous vide. On filtre 63,3 g d'un précipité blanc fondant à 162° C suffisamment pur pour être utilisé par ailleurs.

Rendement : 85%

b) Estérification :

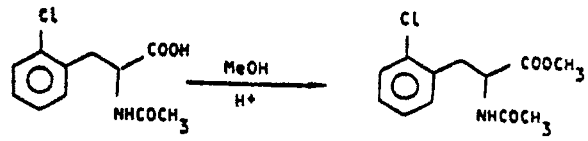


5,4 g d'amido-acide (0,022 M) dissous dans 50 $cm^3$ d'acide sulfurique concentré sont mis à reflux 4 heures. Le mélange est alors dilué dans l'eau. On concentre sous vide, on extrait à l'éther.
Après lavages à la soude et à l'eau, l'évaporation des phases éthérées donne 4,75 g d'une huile, dont les spectres IR et RMN correspondent au produit attendu.
Rendement : 83,3%

c) Hydrolyse enzymatique :

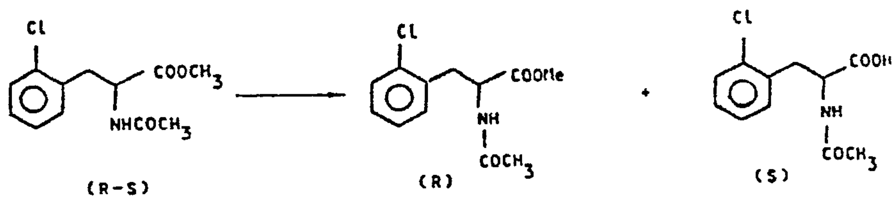


49 g d'amido-ester sont mis en suspension dans 1 l d'eau à pH = 7,5. On ajoute 500 mg de subtilisine sous bonne agitation, le pH est régulé à 7,5 par adjonction de soude 0,2 N. Après 2 heures, le pH est stabilisé. On extrait l'ester au chlorure de méthylène ;
la phase aqueuse est acidifiée à pH = 1 avec $H_2SO_4$, est extrait à l'acétate d'éthyle. On extrait 20 g d'acide et 20 g d'ester.
Rendement : 87%
Les spectres I.R. et RMN sont en accord avec les structures attendues.

ester = + 14,6° acide : - 11,5°

d) Hydrolyse :

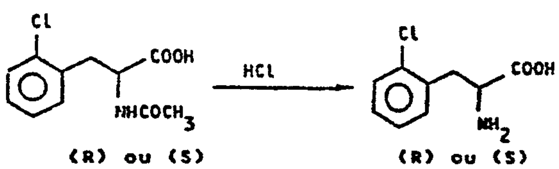


R = H,Me
$[\alpha]_D^{20}$ = - 8,1°
$[\alpha]_D^{20}$ = + 8,1°

L'amido-acide et l'amido-ester sont hydrolysés de manière identique par reflux durant 1 heure dans HCL 5N.
Après refroidissement, le précipité est lavé avec le minimum d'eau, puis repris à l'ammoniaque pour libérer la base. Le précipité blanc obtenu est lavé à l'eau.
Rendement quantitatif.

Stade B

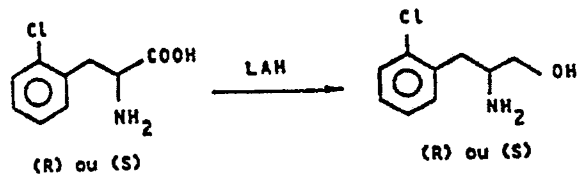


A une suspension de 6,45 g deLiA1H$_4$ (0,169 M ; 2 éq.) dans 500 cm$^3$ de THF sec, on ajoute en refroidissant 16,9 g (0,084 M) d'amino-acide. On chauffe ensuite au reflux 4 heures.

Après refroidissement, le mélange est traité par une bouillie de Na$_2$SO$_4$ ; le solide est filtré et lavé plusieurs fois au méthanol. Après évaporation et traitement acido-basique, les amino-alcools sont suffisamment purs pour être utilisés.

$$\left[\alpha_D^{20}\right] = +\ 18°\qquad\qquad \left[\alpha_D^{20}\right] = -\ 15,15°$$

<u>Stade</u> <u>C</u>

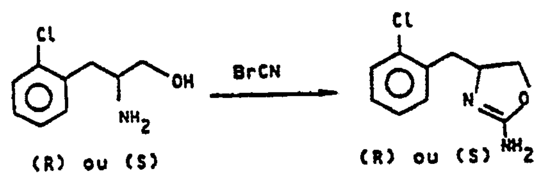


La cyclisation s'effectue comme déjà décrit pour les composés racémiques.
Les anentiomères obtenus présentent les pouvoirs rotatoires suivants

isomère dextrogyre $\left[\alpha_D^{20}\right] = +\ 0,85°$

isomère levogyre $\left[\alpha_D^{20}\right] = -\ 10°$

## ETUDE PHARMACOLOGIQUE DES COMPOSES SELON L'INVENTION

La recherche de l'activité pharmacologique des composés selon l'invention a abouti à la découverte d'un ensemble de propriétés intéressants :

1) les récepteurs adrénergiques centraux et périphériques.
2) L'appareil cardiovasculaire.
3) Le système nerveux central.

### 1) ACTIVITE VIS-A-VIS DES RECEPTEURS ADRENERGIQUES

Chez le lézard (anolis carolinensis), il y a une relation à forte corrélation entre les récepteurs $\alpha_2$ et l'hormone MSH agissant au niveau des mélanocytes de la peau. Le noircissement, qui résulte du traitement par le MSH, est antagonisé par les $\alpha_2$ agonistes (clonidine), alors que les $\alpha_2$ antagonistes induisent eux-mêmes le phénomène (Carter and Shuster, Br. J. Pharmacol. (1982), 75, 169-176).

Les essais effectués dans notre laboratoire aboutissent au même résultat, le guppy étant soumis à l'action du méclofénoxate (Lucidril), substance connue comme agissant sur l'axe hypothalamo-hypophysaire. Le noircissement du poisson ainsi provoqué est antagonisé par les $\alpha_2$ agonistes, comme la clonidine.

Les essais réalisés à partir des composés selon l'invention ont montré deux sortes d'activités :

1) Certaines des molécules (composés des exemples 11,22,23,24 ont une activité comparable à celle de la clonidine ($\alpha_2$ agoniste), c'est-à-dire qu'elles antagonisent le noircissement provoqué par le méclofénoxate.

2) D'autres molécules (composés des exemples 6 et 19) ont une activité comparable à celle des antagonistes $\alpha_2$ , comme la yohimbine, et provoquent spontanément le noircissement

2) ACTIVITE CARDIOVASCULAIRE CHEZ LE CHIEN ANESTHESIE

Chez le chien anesthésié, l'injection des produits selon l'invention provoque une hypertension durable, à des doses relativement faibles, allant de 0,05 à 1 mg/kg I.V.

L'activité tensive est associée selon le cas, à une vasodilatation primaire de courte durée, suivie d'un état de vasoconstriction plus ou moins durable, ou à une vasoconstriction primaire durable. Parmi les substances possédant une activité du premier type (vasodilatation primaire), on note le composé de l'exemple 2 qui montre, par ailleurs, un effet antagoniste de la vasoconstriction de l'artère mésentérique à la dopamine (effet connu comme étant de type $\alpha_2$ adrénergique), ce qui confirme l'origine du noircissement de ces substances au niveau des mélanocytes.

Parmi les substances induisant une activité vasoconstrictive d'emblée (baisse des débits artériels fémoraux), on note, par exemple les composés des exemples et la clonidine.

3) ACTIVITE AU NIVEAU DU SYSTEME NERVEUX CENTRAL CHEZ LA SOURIS

L'activité des produits a été recherchée sur différents tests pharmacologiques :

1) Activité sur la température centrale chez la souris
2) Activité antidépressive chez la souris :
   a) hypothermie à la réserpine
   b) hypothermie à l'apomorphine
   c) toxicité à la yohimbine
3) Activité motrice chez la souris.

1°) ACTIVITE SUR LA TEMPERATURE CENTRALE CHEZ LA SOURIS

L'administration des produits selon l'invention aboutit à une baisse de température des animaux, à l'exemple de la clonidine, à des doses allant de 0,75 à 20 mg/kg. Ces substances ont des propriétés comparables à celles de la yohimbine chez le poisson et chez le chien anesthésié. Ceci confirme l'identité d'action entre ces substances et la clonidine au niveau des récepteurs $\alpha_2$.

2°) ACTIVITE ANTIDEPRESSIVE CHEZ LA SOURIS

Certaines substances des composés selon l'invention présentent des propriétés antidépressives sur la plupart des tests cités :

| | HYPOTHERMIE RESERPINE | HYPOTHERMIE APOMORPHINE | TOXICITE YOHIMBINE |
|---|---|---|---|
| Composé N° 2 | 0 | + | + |
| " " 9 | + | + | + |
| " " 12 | 0 | + | + |
| " " 14 | + | + | + |
| " " 16 | + | + | 0 |
| " " 18 | + | + | - |

3°) ACTIVITE MOTRICE CHEZ LA SOURIS

A l'exception du composé de l'exemple 18 , toutes les substances ayant une activité centrale ou antidépressive et/ou ayant une activité antagoniste ou agoniste sur les récepteurs 2 diminuent l'exploration chez la souris, à l'exemple de la clonidine.

4) ACTIVITES ANNEXES

1°) ACTIVITE DIURETIQUE CHEZ LA SOURIS

Quelques molécules ont une activité diurétique, cette activité restent dans l'ensemble modérée. La clonidine peut exercer elle-même des activités diurétiques, mais dans cette série, il n'y a pas de corrélation entre les propriétés $\alpha_2$ agonistes et diurétiques.

2°) ACTIVITE DIABETOGENE

Seules les substances ayant une activité centrale comparable à celle de la clonidine ont, comme elle, une activité diabétogène expérimentale.

DISCUSSION ET CONCLUSION

Le spectre d'activité pharmacologique des composés selon l'invention peut être résumé comme suit :

1) Une activité antidépressive puissante observée sur la plupart des tests pharmacologiques.

2) Une activité $\alpha_2$ agoniste de type clonidine, suggérant une possible action dans l'hypertension.

3) Une activité $\alpha_2$ antagoniste qui, liée à une activité antidépressive, peut permettre l'obtention, en thérapeutique, d'une amélioration plus rapide de l'état dépressif.

ETUDE BIOLOGIQUE DES COMPOSES SELON L'INVENTION

ACTIVITE DES PRODUITS SUR LES TESTS D'INHIBITION D'UPTAKE DES NEUROTRANSMETTEURS ET D'AFFINITE SUR LE RECEPTEUR $\alpha_2$ "IN VITRO"

| PRODUITS | UPTAKE NEUROTRANSMETTEUR IN VITRO IC 50 M | | | AFFINITE RECEPTEURS $\alpha_2$ IN VITRO IC 50 M |
|---|---|---|---|---|
| | NAd | 5-HT | DA | |
| DESIPRAMINE | $1,3 \times 10^{-8}$ | | | |
| IMIPRAMINE | | $5,7 \times 10^{-7}$ | | |
| DOPAMINE | | | $8,0 \times 10^{-7}$ | |
| PHENTOLAMINE | | | | $2,2 \times 10^{-8}$ |
| CLONIDINE | | | | $10^{-9}$ |
| IDAZOXAN | | | | $2,9 \times 10^{-8}$ |
| ex. 5 | $> 10^{-5}$ | $2,8 \times 10^{-6}$ | $7,2 \times 10^{-6}$ | $3,3 \times 10^{-8}$ |
| ex. 6 | nul à $10^{-5}$ | $> 10^{-5}$ | $> 10^{-5}$ | $1,5 \times 10^{-7}$ |
| ex. 7 | nul à $10^{-5}$ | $> 10^{-5}$ | nul à $10^{-5}$ | $> 10^{-7}$ |
| ex. 8 | nul à $10^{-5}$ | $> 10^{-5}$ | nul à $10^{-5}$ | $> 10^{-7}$ |
| ex. 9 | $1,2 \times 10^{-6}$ | $5,3 \times 10^{-5}$ | $8,3 \times 10^{-6}$ | $3,6 \times 10^{-8}$ |
| ex. 10 | $4,0 \times 10^{-6}$ | $4,0 \times 10^{-6}$ | $2,3 \times 10^{-6}$ | $4,4 \times 10^{-8}$ |
| ex. 11 | $2,5 \times 10^{-6}$ | $> 10^{-5}$ | $> 10^{-5}$ | $2,7 \times 10^{-9}$ |
| ex. 12 | $4,0 \times 10^{-7}$ | $> 10^{-5}$ | $4,0 \times 10^{-6}$ | $4,0 \times 10^{-9}$ |
| ex. 13 | $2,3 \times 10^{-7}$ | nul à $10^{-6}$ | nul à $10^{-6}$ | $> 10^{-7}$ |
| ex. 14 | $10^{-6}$ | nul à $10^{-6}$ | nul à $10^{-6}$ | $4,0 \times 10^{-8}$ |
| ex. 15 | $10^{-6}$ | nul à $10^{-6}$ | nul à $10^{-6}$ | $3,7 \times 10^{-9}$ |
| ex. 16 | | | | $> 10^{-7}$ |
| ex. 17 | | | | $> 10^{-7}$ |
| ex. 18 | | | | $1,5 \times 10^{-9}$ |
| ex. 20 | | | | $> 10^{-7}$ |
| ex. 21 | | | | $1,9 \times 10^{-9}$ |
| ex. 22 | | | | $2,7 \times 10^{-9}$ |
| ex. 23 | | | | $3,3 \times 10^{-9}$ |
| ex. 24 | | | | $1,5 \times 10^{-8}$ |

NAd = Noradrénaline
5-HT = 5-hydroxytryptophane
DA = Dopamine

## Revendications

1. Les 2-amino oxazolines de formule générale I

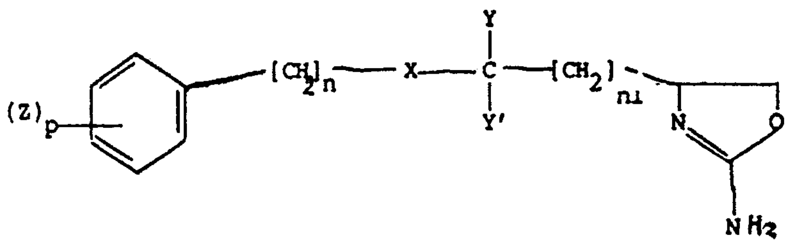

(I)

dans laquelle Z est de l'hydrogène, un atome d'halogène, un radical alcoxy ayant de 1 à 6 atomes de Carbone
X représente de l'oxygène, du soufre, un radical imino, un radical méthylène ou une liaison directe
Y représente de l'hydrogène
Y' représente de l'hydrogène
ou bien Y et Y' forment ensemble l'oxygène d'un groupe carbonyle
ou bien Y' forme avec le radical phényle adjacent lorsque n est égal à zéro, une structure bicyclique, hétérocyclique, saturée ou non saturée, choisie dans le groupe constitué par le benzofuran, l'indole, l'indoline et le dihydrobenzofuran

n est égal à zéro ou 1
$n_1$ est égal à O, 1 ou 2
et p est égal à 1, 2 ou 3
sous forme racémique ou optiquement active

2. Les sels des composés selon la revendication 1° avec un acide minéral ou organique.

3. Les isomères optiquement-actifs des composés selon l'une des revendications 1° ou 2°.

4. Un composé selon la revendication 1°, à savoir la 4-phénoxyméthyl 2-amino oxazoline.

5. Un composé selon la revendication 1° ou la revendication 2°, à savoir la 4-benzyloxyméthyl 2-amino oxazoline et son chlorhydrate.

6. Un composé selon la revendication 1° ou la revendication 2°, à savoir la 4-[(2-chlorophényl)méthyl oxyméthyl] 2-amino oxazoline et son chlorhydrate.

7. Un composé selon la revendication 2°, à savoir la 4-[(2,6-dichlorophenyl) methyloxymethyl] 2-amino oxazoline et son chlorhydrate.

8. Un composé selon la revendication 1° ou la revendication 2), à savoir la 4-[(2,3-dichloro 4-méthoxyphényl) méthyl] 2-amino oxazoline et son chlorhydrate.

9. Un procédé d'obtention des composés de formule I selon la revendication 1° qui consiste en ce que l'on réduit un dérivé aminé de formule générale II racémique ou optiquement-actif

(II)

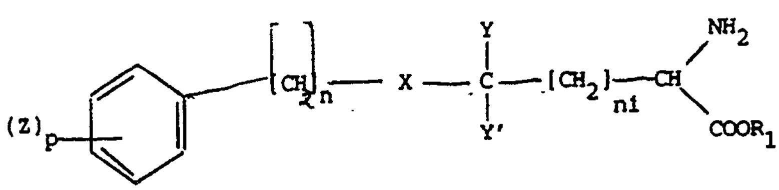

dans laquelle les substituants Z, p, n, X, Y, Y' et $n_1$ sont définis comme à la revendication 1°
et $R_1$ est un radical alcoyle inférieur ou de l'hydrogène par un hydrure mixte de métal alcalin, en un amino-alcool de formule générale III

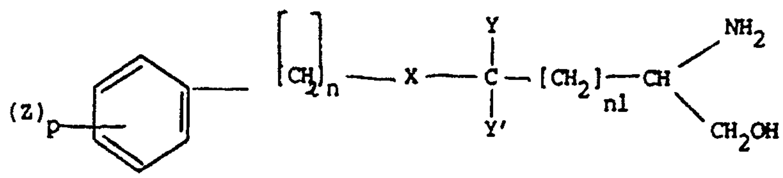

(III)

sous forme racémique ou optiquement-active dans laquelle les substituants Z, p, n, X, Y, Y' et $n_1$ sont définis comme à la revendication 1°, puis par action d'un halogénure de cyanogène en présence d'un agent alcalin, le transforme en une amino-oxazoline de formule I sous forme racémique ou optiquement-active.

## Claims

1. The 2-amino oxazolines having the general formula I

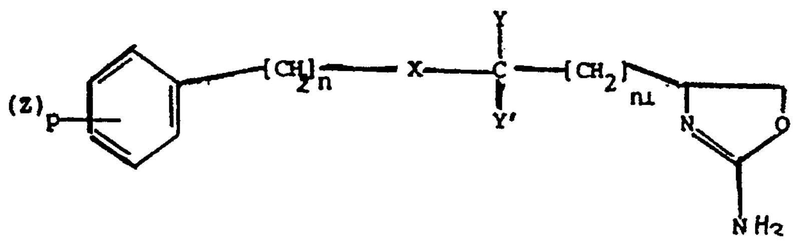

(I)

wherein Z is a hydrogen, a halogen atom, an alkoxy radical having from 1 to 6 carbon atoms.
X is an oxygen, a sulphur, an imino radical, a methylen radical or a direct link
Y is a hydrogen
Y' is a hydrogen
or Y and Y' together form the oxygen of a carbonyl group.
or Y' forms with the adjacent phenyl ring, when n is zero, a bicyclic, heterocyclic, unsaturated or saturated structure selected from the group consisting of benzofuran, indole, indoline and dihydrobenzofuran.
n is equal to zero or 1
$n_1$ is equal to zero, 1 or 2
and p is equal to 1, 2 or 3
in the racemic or optically-active form.

2. The salts of the compounds according to claim 1° with a mineral or organic acid.

3. The optically-active isomers of the compounds according to any of the claims 1 or 2.

4. A compound according to claim 1°, i.e. 4-phenoxymethyl 2-amino oxazoline.

5. A compound according to claim 1° or claim 2° i.e. 4-benzyloxymethyl 2-amino oxazoline and its hydrochloride.

6. A compound according to claim 1° or claim 2°, i.e. 4-[(2-chlorophenyl)methyl oxymethyl] 2-amino oxazoline and its hydrochloride.

7. A compound according to claim 2°, i.e. 4-[(2,6-dichlorophenyl) methyloxymethyl) 2-amino oxazolin and its hydrochloride.

8. A compound according to claim 1° or claim 2°, i.e. 4-[(2,3-dichloro 4-methoxyphenyl) methyl] 2-amino oxazoline and its hydrochloride.

9. A process for producing the compounds of formula I according to claim 1° which consists in that an amino derivative of general formula II in the racemic or optically-active form

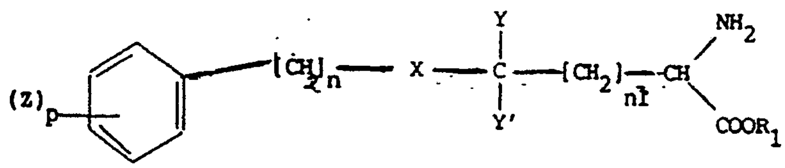

(II)

wherein the definitions of the substituents Z, p, n, X, Y, Y' and $n_1$ are defined as in claim 1°
and $R_1$ is a lower alkyl radical or a hydrogen
is reduced by means of an alkali metal mixed hydride, into an amino-alkanol of general formula III

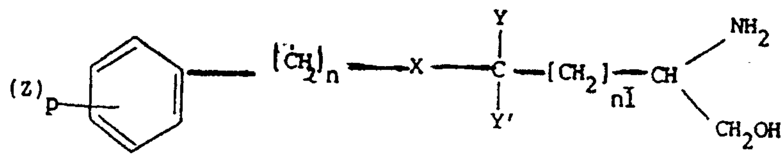

(III)

in the racemic or optically-active form
in which the substituents Z, p, n, X, Y, Y', and n1 are defined as in claim 1°
then transforms it into an amino oxazoline of formula I, by means of a cyanogen halide in the presence of a basic agent, in the racemic or optically-active form.

**Patentansprüche**

1. Die neue 2-Amino oxazoline der allgemeinen Formel I

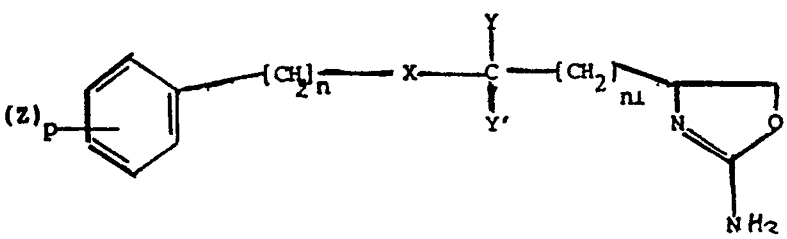

(I)

worin Z ein Wasserstoff atom, ein Halogen atom, ein $C_1$ - $C_6$ alkoxy Rest ist,
X fur ein Sauerstoff, ein Schwefelatom, ein Imino gruppe, ein Methylen rest, oder eine direkte Bindung steht
Y ein Wasserstoff atom ist,
Y' ein Wasserstoff atom ist,
oder Y und Y' zusammen das Sauerstoff atom einer Karbonyl gruppe bilden
oder Y' mit der nachbarten Phenyl rest, wenn n gleich Null ist,
eine bicyclische-heterocyclische, gesättigte oder ungesättigte Ring Struktur bildet der aus der gruppe von Benzofuran, Indol, Indolin, und Dihydrobenzofuran gewählt ist
n gleich Null oder 1 ist
$n_1$ gleich Null, 1, oder 2 ist
und p gleich 1, 2, oder 3 ist
in razemischen oder optisch-aktiven Form.

2. Die Salze der Verbindungen nach Anspruch 1° mit einer anorganischen oder organischen Säure.

3. Die optisch-aktive Isomere der Verbindungen nach einer der Ansprüche 1 oder 2

4. Eine Verbindung nach Anspruch 1 d.h. 4-phenoxymethyl 2-amino oxazolin.

5. Eine Verbindung nach Anspruch 1° oder Anspruch 2° d.h. 4-benzyloxymethyl 2-amino oxazolin und dessen Hydrochlorid.

6. Eine Verbindung nach Anspruch 1° oder Anspruch 2° d.h. 4-[(2-chlorphenyl)methyl oxymethyl] 2-amino oxazolin und dessen Hydrochlorid.

7. Eine Verbindung nach Anspruch 2° d.h. 4-[(2,6-dichlorphenyl)methyoxymety] 2-amino oxazolin und dessen Hydrochlorid.

8. Eine Verbindung nach Anspruch 1° oder Anspruch 2° d.h. 4-[(2,3-dichlor 4-methoxyphenyl)methyl] 2-amino oxazolin und dessen Hydrochlorid.

9. Ein Verfahren zur Herstellung der Verbindungen der allgemeinen Formel I nach Anspruch 1° das darin besteht dass ein amino Derivat der allgemeinen Formel II in der razemischen oder optisch aktiven Form,

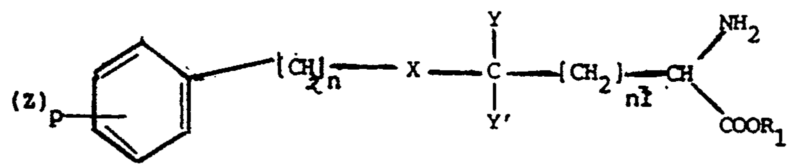

(II)

worin die Substituenten Z, p, n, X, Y, Y' und $n_1$ wie in Anspruch 1° definiert sind und $R_1$ ein niedrig Alkyl Rest oder ein Wasserstoff atom ist mittels eines Alkalimetal misch-hydrid in einem Amino alkanol der allgemeinen Formel III

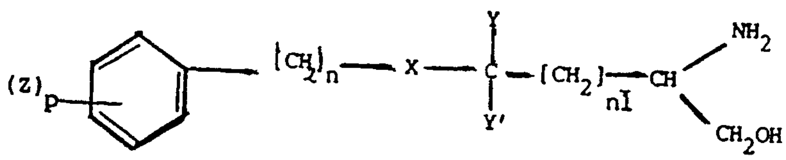

(III)

in der razemischen oder optisch-aktiven Form
worin die Substituenten Z, p, n, X,Y, Y' und $n_1$ die gleiche Bedeutungen wie in Anspruch 1° haben, reduziert wird,
das danach unter die Wirkung eines Cyanogen Halids in Anwesenheit eines alkalischen Mittels in einen Amino oxazolin der Formel I in der razemischen oder optisch-aktiven Form, uberfuhrt.